# EUROPÄISCHE PATENTANMELDUNG

(11) **EP 3 695 816 A1**
(43) Veröffentlichungstag der Anmeldung: **19.08.2020**
(21) Anmeldenummer: 20000067.7
(22) Anmeldetag: 14.02.2020
(51) Int. Cl.: A61F 5/01, A61F 2/50, A61H 3/00

(54) **STÜTZFEDER FÜR DEN EINBAU IN EINE UNTERSCHENKELORTHESE**

(30) Priorität: 14.02.2019 DE 102019001106
(71) Anmelder: Kajamed GmbH, 08056 Zwickau (DE)
(72) Erfinder: Fiedler, Jan, 08056 Zwickau (DE); Ganzer, Thomas, 08056 Zwickau (DE)
(74) Vertreter: Auerbach, Bettina

(57) **Zusammenfassung**

Die Erfindung betrifft eine Stützfeder für den Einbau in eine Unterschenkelorthese, welche einen Sohlenbereich (4), der bis zum Vorderfuß des Trägers der Orthese reicht und die jeweiligen Außenkonturmaße des Fußbereichs des Trägers annähernd erreicht sind, einem Fersenbereich (3), der bis zum Sprunggelenkbereich (2) reicht und als eine der tatsächlichen Fersenform und -größe des Trägers angepasste 3D-Fersenfrom (3.1) ausgebildet ist, einen Sprunggelenkbereich (2), der den Federbereich der Stützfeder bildet und zwischen Sohlenbereich (4) und Sprunggelenkbereich ein Winkel annähernd der Trägergeometrie ausgebildet ist und eine Wadenbereich (1) aufweist, der nach vorn gewölbt ausgebildet ist, einen zusätzlichen Stabilisierungskem (1.1) aufweist und jeweils seitliche, vorgeformt gewölbte Flügelschalen (1.2) angeordnet sind.

Die Vorteile bestehen in der Materialeinsparung, der Gewichtsreduzierung, der geringen Aufbauhöhe im Sohlen- und Fersenbereich. Durch die Verlagerung der Bewegungsachse und des Drehpunktes in den Sprunggelenkbereich ist eine sehr hohe Bewegungsfreiheit für den Träger erreicht.

## Beschreibung

Die Erfindung betrifft eine Stützfeder für den Einbau in eine Unterschenkelorthese mit einem gekrümmten Fersenteil, einem daran weiterverlaufenden fußseitigen Endabschnitt und ein zur anderen Seite weiterverlaufender unterschenkelseitiger Endabschnitt, wobei die Stützfeder aus faserverstärktem Kunststoff besteht. Die Stützfeder soll Anwendung finden bei Patienten mit halbseitiger Lähmung, mit schweren Fußdeformitäten, mit Diparese oder mit Spitzfußgang. Dabei soll die Feder im hinteren Bereich des Unterschenkels und/oder im unteren Bereich des Fußes einer Orthese eingebaut werden.

Lösungen von Stützfedern für eine Fußgelenksorthese sind bereits aus dem Stand der Technik bekannt.
So beschreibt die DE 199 05 544 B4 eine Stützfeder mit einem gegenläufig gekrümmten Fersenteil, über das ein fußseitiger Endabschnitt und ein unterschenkelseitiger Endabschnitt miteinander verbunden sind, die Feder aus einem faserverstärkten Kunststoff hergestellt ist, wobei die Stützfeder einen rechteckigen Querschnitt aufweist und deren Federrate nach dem Gewicht des Patienten ausgelegt ist. Vorteil dieser Lösung ist, dass ein Abwinkeln des Fußes nach oben oder unten bestimmbar freigegeben werden kann. Dabei ist jedoch die Bewegung nach hinten nur in eingeschränktem Umfang möglich, während die Bewegung nach vorn über einen größeren Winkelbereich möglich ist.

Nachteil dieser Lösung ist die festgelegte Querschnittsform der Stützfeder. Dadurch sind die Übergangsbereiche in der Orthese sowohl im Wadenbereich als auch im Fußbereich nur durch einen größeren Aufwand bei der Einbettung in das Material der Orthese in diesem Bereich möglich, um den Patienten einen angenehmen Tragekomfort bieten zu können. Dadurch wird die Orthese jedoch schwerer und optisch klobiger.

Eine weitere technische Lösung wird in der EP 2 568930 B1 beschrieben. Dabei wird der Aufbau eines Stützkörpers aus einem Verstärkungsgebilde, welches aus einer Trägerlage und einer oder mehrerer Gelegeschichten mit Verstärkungsfasern beschrieben, wobei die Verstärkungsfasern aus endlos gelegten Faserbündeln auf der Trägerlage fixiert angeordnet werden und durch nachträgliche Formgebung des mit einer aushärtbaren Grundmasse getränkten Verstärkungsgebildes der Stützkörper gebildet wird. Durch die lösungsgemäße Legung der Endlosfaserbündel werden die angestrebten geschlossenen Randfaserbündel ausgebildet, um die Rissbildung beim Gebrauch der Orthese zu verhindern.
Nachteil dieser Lösung besteht in dem erhöhten Aufwand für die Einstellung der spezifischen Belastbarkeit des Stützkörpers in den verschiedenen Unterschenkel- und Fußbereichen in Bezug auf das Trägerverhalten. Durch die Endlosfasergelege innerhalb des Stützkörpers kann die partielle Stütz- und Federkraft nicht optimal eingestellt werden, da immer die gleiche Anzahl von Verstärkungsfasern innerhalb des Stützkörpers in dessen einzelnen Bereichsabschnitten vorhanden sind. Weiterhin lässt die gezeigte Geometrie der Stützfeder keine Unterstützung des Fersenbereichs zu, was für eine Verlagerung der Bewegungsebene und des Drehpunktes in den Bereich des Sprunggelenkes jedoch unbedingt erforderlich ist, um Verletzungsgefahren auszuschließen und eine Unterstützung des Gangverhaltens zu erreichen.

Es ist deshalb Aufgabe der Erfindung, eine technische Lösung zu schaffen, mit deren Hilfe die Nachteile des Standes der Technik überwunden werden.
Es soll eine Stützfeder für Unterschenkelorthesen entwickelt werden, welche in Abhängigkeit von Körpergewicht, Körpergröße und körperlicher Aktivität des Patienten als vorgefertigtes dennoch konfektioniertes Fertigprodukt bereitgestellt werden. Der Einsatz der eingebauten Materialien soll auf ein Minimum reduziert werden, um den Ressourcenverbrauch effektiv zu gestalten und das Gewicht der Stützfeder zu minimieren. Die Stütz- und Federkraft der Stützfeder soll nicht nur durch die Wahl des Materialeinsatzes sondern durch die Gestaltung der geometrische Form der Stützfeder so leistungsorientiert erzielt werden, dass die Hauptbewegung und der Drehpunkt der Stützfeder in den Bereich des Sprunggelenks und nicht in der Ferse verlagert wird. Dadurch soll ein für die Patienten angenehmerer Tragkomfort erreicht werden, welcher zudem ein dynamischeres Gehverhalten für den Patienten zulässt.

Erfindungsgemäß wird die Aufgabe durch die Merkmale des Anspruchs 1 gelöst, wobei die vorteilhaften Ausgestaltungen in den Unteransprüchen beschrieben sind.

Danach besteht die erfindungsgemäße Stützfeder für den Einbau in eine Unterschenkelorthese aus einem gekrümmten Fersenteil, einem daran weiterverlaufenden fußseitigen Endabschnitt als Sohlenbereich und ein zur anderen Seite weiterverlaufendem unterschenkelseitigen Abschnitt im Sprunggelenkbereich und einem weiterverlaufenden Abschnitt im Wadenbereich, wobei die Stützfeder aus faserverstärktem Kunststoff besteht und Verstärkungsstrukturen aufweist.

Erfindungsgemäß ist der Sohlenbereich der Stützfeder bis zum Vorderfuß des Trägers ausgebildet. In diesem Vorderfußbereich ist die Stützfeder breiter ausgebildet und verjüngt sich über den Mittelfußbereich bis hin zum unteren Fersenbereich. Die dadurch erfolgte Formgebung dient als Auflage für den Fuß des Trägers und soll deshalb die jeweiligen Außenkonturmaße des Fußbereichs des Trägers annähernd erreichen. Dadurch wird erreicht, dass die untere Fußsohle des Trägers beim Laufen mit unterstützt wird und eingebunden ist. Der Sohlenbereich kann je nach Bedarf auf die genaue Größe des Trägers gekürzt werden. Diese breite Auflage der Stützfeder verhindert eine unnütze Belastung des mittleren Längsbereiches des Fußes, was bei zu schmalen Orthesen oft der Fall ist.

Zur Stabilisierung des unteren Fußbereiches kann wenigstens eine, im unteren Fersenbereich angeordnete, materialverstärkte Stabilitätssicke geformt ausgebildet. Vorteilhafter Weise können auch mehrere Verstärkungssicken über die Breite des unteren Fersenbereiches angeordnet werden, um die partiell notwenige Materialverstärkungen zu minimieren und besser in diesem Bereich zu verteilen. Damit wird die Druckentlastung auf den Fuß verbessert und die Stützkraft der Stützfeder in diesem Bereich erhöht.

Der sich anschließende Fersenbereich ist bis zum Sprunggelenksbereich der Stützfeder als eine der tatsächlichen Fersenform und -größe des Trägers angepasste 3D-Fersenfrom ausgebildet. Die 3D-Form ist der Form der Ferse in diesem Bereich nachempfunden. Dadurch ist es möglich, dass beim Träger der Orthese seine Ferse in diesem Bereich anliegt und aufgenommen wird. Durch die Möglichkeit der Anordnung von zusätzlichen Stabilisierungssicken im unteren Fersenbereich kann die Aufnahme und Einbettung der Ferse unterstützt werden, da die Stützfeder in diesem Bereich zusätzlich stabilisiert wird. Dies wird auch durch die 3D-Form-Ausbildung des Fersenbereiches unterstützt, da auch hier die Flexibilität der Stützfeder weitestgehend eingeschränkt ist.

Die 3D-Form-Ausbildung des Fersenbereiches bietet weiterhin den Vorteil, dass die Ferse des Trägers der Orthese auch beidseitig stabilisiert wird. Ein weiterer Vorteil dabei ist, dass in diesem Fersenbereich keine zusätzliche Materialanhäufung notwendig ist, um die notwendige Stabilisierung zu erreichen, sondern allein durch die 3D-Formgebung wird die Stabilisierung erreicht.
Um Material einzusparen, das Gewicht zu reduzieren und den Tragekomfort zu erhöhen, weist die 3D-Fersenform eine Ausnehmung auf, welche annähernd ein Drittel der Fersenformgröße des Trägers aufweist. Die Form kann dabei oval, rund oder als ein oder mehrere Schlitze ausgeführt sein.

Mit dieser Ausnehmung werden auch die im Fersenbereich auftretenden Biegekräfte aufgenommen und in diesem Fersenbereich verteilt in den Bereich der Ausnehmung abgeleitet, so dass es zu keinen Rissbildungen durch Beanspruchungen im äußeren Kantenbereich kommt.

Zum Stützen des Unterschenkels ist die Anordnung des Sprunggelenkbereichs an den Fersenbereich so ausgebildet, dass zwischen Sohlenbereich und Sprunggelenksbereich ein Winkel, annähernd der Geometrie des Trägers nachempfunden, ausgebildet ist.

Der Sprunggelenksbereich ist als der hauptsächliche Federbereich ausgebildet und weist aus diesem Grund keine zusätzlichen Stabilisierungsmaßnahmen auf. Dieser Bereich ist wie der Sohlenbereich aus dem faserverstärkten Kunststoff ausgebildet.
An den Sprunggelenksbereich schließt sich der Wadenbereich an, welcher nach vorn gewölbt ausgebildet ist und einen zusätzlichen Stabilisierungskern aufweist. Im oberen Endbereich sind jeweils seitlich und der Wadenform und - größe des Trägers angepasste, vorgeformt gewölbte Flügelschalen angeordnet, welche allein durch diese Schalenform eine zusätzlichen Stabilisierungsmaßnahme bilden.
Mit dieser geometrischen 3D-Gestaltung und unterschiedlichen Anordnung von Stabilisierungsmaßnahmen im Verlauf der Stützfeder wird die angestrebte vorteilhafte Verlagerung der Bewegungsachse und des Drehpunktes der Stützfeder in den Sprunggelenksbereich erreicht und bildet damit die Voraussetzung für die Optimierung des Bewegungsablaufes und des Gangverhaltens und somit zur Erhöhung des Tragekomforts.

Unterstützt wird diese Maßnahme durch den gewählten Materialeinsatz. Erfindungsgemäß weist die Stützfeder ein textiles Halbzeug auf, welches laminatartig in einem unter Druck und bei gleichzeitiger Formgebung ausgehärteten Kunstharz oder Kunststoff eingebettet ist. Der so gebildete Faser-Kunststoff-Verbund weist somit in allen Bereichen die verstärkungsfasern auf. Diese gleichmäßige Anordnung der leistungs- und bedarfsgerecht angeordneten Verstärkungsfasern auf der Trägermatte wird mit geeigneten technologischen Mitteln, wie Kleben oder aufnähen, fixiert, bevor die Einbettung des textilen Halbzeuges in einen Kunststoff erfolgt.
Die angeordneten zusätzlichen Verstärkungsfasern auf der Trägermatte bestehen wie die Trägermatte selbst aus organischen, anorganischen und/oder Naturfasern. Vorzugsweise werden Carbonfasern, Glasfasern, Mineralfasern, Naturfasern oder eine Kombination aus diesen Faserarten eingesetzt. Die auf der Trägermatte angeordneten Verstärkungsfasern können aber auch mit schmelzbaren Materialien vorimprägniert werden, welche dann thermisch miteinander verschmelzen. Die Abstimmung des Fasereinsatzes in Qualität und Menge wird für das Vorkonfektionieren der Stützfeder jeweils bedarfsgerecht ausgewählt.
Die Einbettung der mit Verstärkungsfasern zusätzlich und bedarfsgerecht ausgerüsteten Trägermatte, welche bereist die Grunddimensionierung der Stützfeder ausweist, in den härtbaren Kunststoff, beispielsweise Epoxidharz, erfolgt unter dreidimensionaler Formgebung, so dass die Querschnittsform der Stützfeder partielle Aufwölbungen und einen 3D-geformten Fersenbereich aufweist. Vorteilhafter Weise sind die Stabilisierungsmaßnahmen nur dort angeordnet, wo das Erfordernis besteht, wie im Wadenbereich und im unteren Fersenbereich, eventuell bis hin zum Sohlenbereich.

Vorteilhafter Weise wird die Querschnittsform zu beiden Enden der Stützfeder verringert, in dem die Materialstärke der Stützfeder zu den beiden Enden -Spitze des Sohlenbereiches und oberer Wadenbereich - sich allmählich verjüngend ausgebildet ist.

Der Endabschnitt im Sohlenbereich der Stützfeder wird vorteilhafter Weise zur Verbesserung der Vorschwungphase bis zu den Zehenspitzen ausgebildet. Dabei ist der Endabschnitt lang, gerade und eben ausgeführt und kann im Bedarfsfall gekürzt werden, um auf die Größe des Fußes und die Bedürfnisse und den Wunsch des Trägers angepasst werden zu können.
Das bietet den Vorteil, dass die erfindungsgemäße Stützfeder in der Vorschwungphase aufrichtend wirkt und die fehlende Kraft im Vorderfuß ersetzt. Bei Stützfedern nach dem bekannten Stand der Technik endet die Feder im Mittelfußbereich, sodass die Patienten, die keine Kraft mehr im Vorderfuß haben, nach vorn wegknicken und sich nicht aufrichten können.

Dadurch kann auf die sonst übliche Materialanhäufung zu Stabilitätszwecken in diesem Bereich verzichtet werden. Dies ist jedoch nur möglich, weil die Trägermatte mit den Verstärkungsfasern für die Einstellung der Federfunktion ausgestattet wird und im Laminier-Verfahren dieser so gestaltete spezielle Faser-Kunststoff-Verbund eine 3-D-Verformung erfährt.

Dabei sind die eingesetzten Verstärkungsfasern und die Fasern der Trägermatte aus anorganischen, organischen und/oder Naturfasern oder einer Kombination aus diesen Fasern. Die Fasern können vorteilhafter Weise Carbonfasern, Glasfasern und/oder Mineralfasern sein.
Eine mit diesen Merkmalen ausgestattete Stützfeder für den Einbau in eine Unterschenkelorthese bietet einerseits die konventionelle Vorfertigung von individuell eingestellten und in ihrer Grundform annähernd für den Einsatz angepassten Stützfedern, welche sich durch ein geringes Gewicht, geringen Stärken in den Übergangsbereichen zur Orthese, der vorteilhaften Federkrafteinstellung im Sprunggelenksbereich, sparsamen und ziel- und aufgabenausgerichteten Materialeinsatz und eine ästhetische Formgebung sowie einen erhöhten Tragekomfort ohne Einschränkung der Bruchsicherheit auszeichnet.
Durch die geringere Aufbauhöhe und die auslaufenden Enden ist die Feder optisch ansprechender und kann durch den sanfteren Übergang optimal in die Orthese eingefügt werden. Durch die veränderte Geometrie und die Gestaltung des angestrebten Kraftaufnahmeverlaufs innerhalb der Stützfeder, die Verlagerung der Bewegungsachse und des Drehpunktes in den Bereich des Sprunggelenkes, lässt diese Stützfeder die Auslenkung eines kompletten Gangzyklus zu. Die Auslenkung nach vorn ist bis zu mindestens 15° und nach hinten bis zu mindestens 5° möglich. Die aus dem Stand der Technik bekannten Federn haben einen deutlich geringeren Bewegungsumfang, und das bei größerer Materialdicke.
Im Vergleich zu bisherigen Produkten bietet die erfindungsgemäße Stützfeder einen langen, geraden und ebenen Endabschnitt, der bis zu den Zehenspitzen reicht.

Das bietet den Vorteil, dass die Feder in der Vorschwungphase (Gangphase) aufrichtend wirkt und die fehlende Kraft im Vorderfuß kompensiert wird.

Die Erfindung soll nachstehend an einem Ausführungsbeispiel erläutert werden. Dabei zeigen:
- Fig. 1: eine schematische Darstellung der Stützfeder mit Sohlenbereich und daran angeordneten Stabilitätssicken, dem 3D-geformten Fersenbereich mit Ausnehmung, dem Sprunggelenkbereich als Federbereich und dem Wadenbereich mit Stabilisierungskern und Seitenflügelanordnung;
- Fig. 2: eine schematische Seitenansicht der Stützfeder von links;
- Fig. 3: eine schematische Unteransicht der Stützfeder;
- Fig. 4: eine schematische Draufsicht der Stützfeder

### Ausführungsbeispiel:

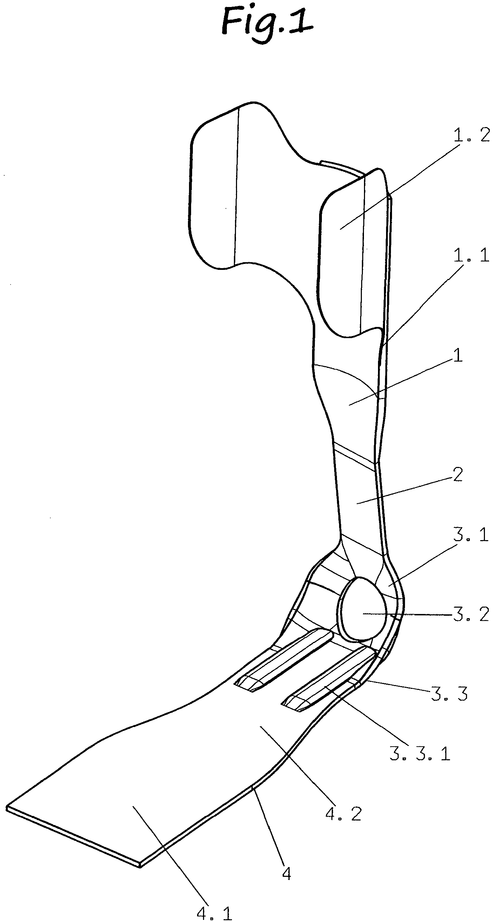

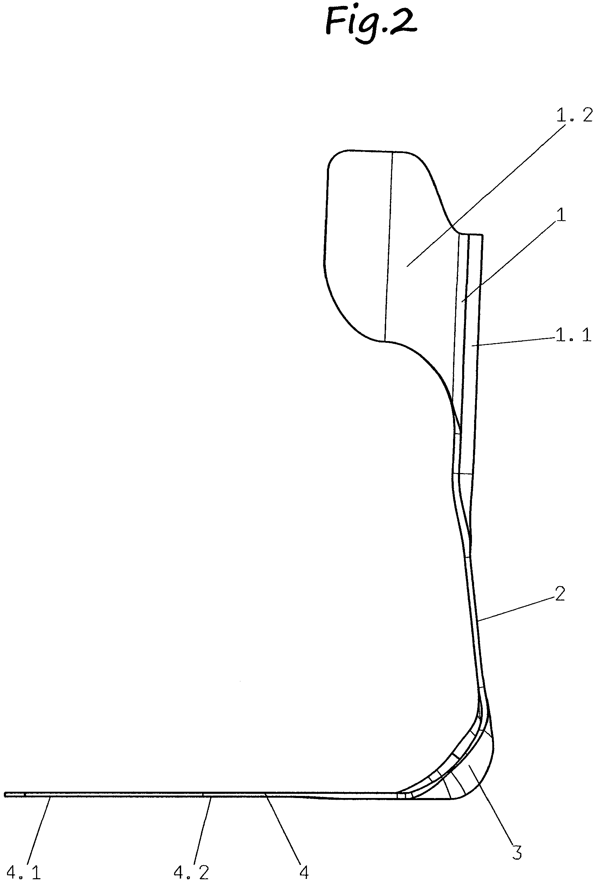

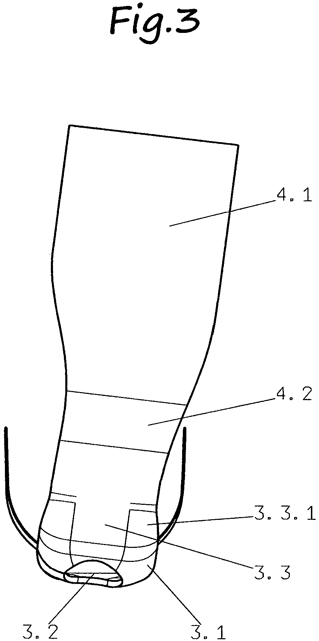

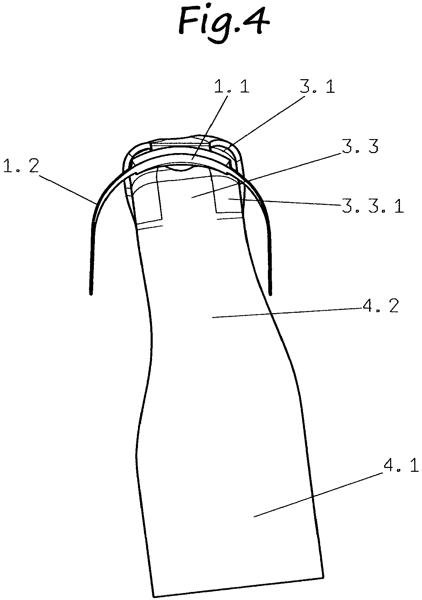

Gemäß der Figuren 1 bis 4 besteht die erfindungsgemäße Stützfeder für den Einbau in eine Unterschenkelorthese aus einem gekrümmten Fersenbereich 3, einem daran weiterverlaufenden fußseitigen Endabschnitt als Sohlenbereich 4 und ein zur anderen Seite weiterverlaufenden unterschenkelseitiger Endabschnitt als Sprunggelenkbereich 3 sowie einen weiterverlaufenden Wadenbereich 1. Diese Bereiche stellen auch gleichzeitig die vier wichtigsten Beanspruchungszonen innerhalb einer Stützfeder für eine Unterschenkelorthese dar.

Auf Grund dieser unterschiedlichen Beanspruchungszonen beim Tragen der Orthese muss die Beschaffenheit und Gestaltung der Stützfeder als Basis für die Orthese auf diese in den einzelnen Bereichen individuell eingestellt und angepasst sein.
Aus diesem Grund weist die Stützfeder im Sohlenbereich 4, welcher bis zu den Fußzehen des Vorderfußes des Trägers der Orthese reicht, in diesem Vorderfußbereich 4.1 eine breiter Ausbildung auf und verjüngt sich über den Mittelfußbereich 4.2 bis hin zum unteren Fersenbereich 3.3 so weit, dass die jeweiligen Außenkonturmaße des Trittflächenbereiches einschließlich der Fußzehen des Trägers annähern erreicht sind. Der Vorderfußbereich 4.1 kann bei Bedarf auf das tatsächliche oder gewünschte Längenmaß des Trägerfußes nachträglich gekürzt werden.
In den beiden Außenbereichen des unteren Fersenbereiches 3.3 sind vorteilhafter Weise zwei Stabilitätssicken 3.3.1 parallel zueinander sowie längs zum Mittelfußbereich 4.2 hin verlaufend angeordnet. Die Stabilisierungssicken können entweder durch 3D-Verfomung oder durch Materialverstärkungen ausgebildet sein und bestehen aus dem gleichen Faser-Kunststoff-Verbund, wie der Rest der Stützfeder.
Der sich an den Sohlenbereich 4 unmittelbar anschließende Fersenbereich 3 ist bis zum Sprunggelenkbereich 2 als eine der tatsächlichen Fersenform und -größe des Trägers angepasste 3D-Fersenfrom 3.1 ausgebildet. Im Mittelpunkt der 3D-Fersenform 3.1 ist vorteilhafter Weise zur Erhöhung des Tragekomforts eine annähern kreisrunde Ausnehmung angeordnet, welche in ihrer Größe annähern ein Drittel der Fersenformgröße der 3D-Fersenform 3.1 aufweist. Dadurch wird nicht nur Material eingespart und das Gewicht der Stützfeder reduziert, sondern auch die Möglichkeit von Druckstellen in diesem Bereich beim Träger wird eliminiert.

An den Fersenbereich 3 schließt sich der Sprunggelenkbereich 2 so an, dass zwischen Sohlenbereich 4 und Sprunggelenkbereich 2 ein Winkel annähernd der Geometrie des Trägers, ausbildet ist.

Der Sprunggelenkbereich 2 ist als Federbereich ohne zusätzliche Stabilisierungsmaßnahmen ausgebildet. Er besteht ebenfalls aus dem gleichen Grundmaterial, wie die Restbereiche der Stützfeder. Dadurch besitzt der Sprunggelenkbereich 2 eine sehr gute Federkraft und genügend Flexibilität, um die Bewegungsabläufe beim Tragen der Orthese zuzulassen. Dies unterstützt wiederum den Muskelerhalt und -aufbau beim Träger.
Der sich an den Sprunggelenkbereich 2 anschließende Wadenbereich 1 ist nach vorn gewölbt ausgebildet und weist einen zusätzliche Stabilisierungskern 1.1. auf. Dieser Kern weist zur Ausbildung einer zusätzlichen Stabilisierung aus dem eingesetzten Faser-Kunststoff-Verbund gebildete Verstärkungsmaßnahmen durch Materialanhäufung oder spezielle Formgebung in diesem Bereich auf und ist ebenfalls eingebettet. Er kann auch durch zusätzliche Einlagerungen von Stützgebilden eingebettet und ausgebildet werden. Dadurch wird der gesamte Unterschenkel gut stabilisiert und mit Hilfe der jeweils seitlich weitestgehend flexiblen und der Wadenform und -größe des Trägers angepassten, vorgeformt gewölbten Flügelschalen 1.2 die Wade sehr gut und stabil aufgenommen.
Als textiles Halbzeug für die Stützfeder wird eine biegeschlaffe textile ebenflächige Trägermatte eingesetzt, auf dem gleichmäßig zusätzliche Verstärkungsfasern mit geeigneten technologischen Mitteln, wie Kleben oder Nähen, fixiert werden, bevor die Einbettung des textilen Halbzeuges in einen Kunststoff erfolgt.

Die Trägermatte, wie auch die auf ihr angeordneten zusätzlichen Verstärkungsfasern bestehen aus anorganischen, organischen und/oder Naturfasern oder einer Kombination aus diesen Fasern. Vorzugsweise werden Carbonfasern, Glasfasern, Mineralfasern, oder Naturfasern sowie eine Kombination aus diesen Faserarten eingesetzt.
Dieses textile Halbzeug wird in diesem Beispiel anschließend mit einer Harzinfusion mittels Unterdruckverfahren und bei gleichzeitiger Formgebung in den ausgehärteten Kunstharz eingebettet. Dabei kann das textile Halbzeug für den Faser-Kunststoff-Verbund auch mehrlagig eingesetzt werden.

Die Vorteile dieser Stützfeder für den Einbau in eine Unterschenkelorthese bestehen zusammengefasst darin, dass die gesamte Feder aus einem einheitlichen zuvor festgelegten Faserkunststoffverbund besteht und lediglich im Bereich der Stabilisierungssicken 3.3.1 und im Stabilisierungskern 1.1 im Wadenbereich 1 zusätzliche Stabilisierungen vorgenommen werden. Mit der Ausbildung von nicht zusätzlich versteiften Bereichen und Stützbereichen innerhalb einer Stützfeder kann der Tragekomfort einer Orthese mit einer derartigen Stützfeder wesentlich erhöht werden, die Muskelkraft des Trägers erhalten und trainiert werden, ohne dabei auf die Sicherheit für den Träger verzichten zu müssen. Die Bewegungseinschränkung wird minimiert. Es wird Material eingespart, das Gewicht der Stützfeder minimiert, die Feder besitzt eine geringe Aufbauhöhe im Sohlen- und Fersenbereich und durch die Verlagerung der Bewegungsachse und des Drehpunktes in den Sprunggelenkbereich eine sehr hohe Bewegungsfreiheit für den Träger. Die Einbindung in den Bau der Orthese ist durch die erreichte Geometrie ebenfalls einfacher.

### Bezugszeichenliste

- 1: Wadenbereich
- 1.1: Stabilisierungskern
- 1.2: Flügelschalen
- 2: Sprunggelenkbereich
- 3: Fersenbereich
- 3.1: 3D-Fersenform
- 3.2: Ausnehmung
- 3.3: unterer Fersenbereich
- 3.3.1: Stabilisierungssicke
- 4: Sohlenbereich
- 4.1: Vorderfußbereich
- 4.2: Mittelfußbereich

## Patentansprüche

1. Stützfeder für den Einbau in eine Unterschenkelorthese mit einem gekrümmten Fersenbereich, einem daran weiterverlaufenden fußseitigen Endabschnitt als Sohlenbereich und ein zur anderen Seite weiterverlaufenden unterschenkelseitigen Endabschnitt im Sprunggelenkbereich und einen weiterverlaufenden Wadenbereich, wobei die Stützfeder aus faserverstärktem Kunststoff mit partiell angeordneten Verstärkungsbereichen besteht, wobei die Fasern in einem ausgehärteten Kunststoff eingebettet sind, **dadurch gekennzeichnet,**
**dass** der Sohlenbereich (4) bis zum Vorderfuß des Trägers der Orthese reicht, in diesem Vorderfußbereich (4.1) breiter ausgebildet ist und sich über den Mittelfußbereich (4.2) bis hin zum unteren Fersenbereich (3.3) so weit verjüngt, dass die jeweiligen Außenkonturmaße des Fußbereichs des Trägers annähernd erreicht sind,
**dass** der Fersenbereich (3) bis zum Sprunggelenkbereich (2) als eine der tatsächlichen Fersenform und -größe des Trägers angepasste 3D-Fersenfrom (3.1) ausgebildet ist,
**dass** sich an den Fersenbereich (3) der Sprunggelenkbereich (2) so anschließt, dass zwischen Sohlenbereich (4) und Sprunggelenkbereich ein Winkel annähernd der Geometrie des Trägers ausgebildet ist,
**dass** der Sprunggelenkbereich (2) als Federbereich ohne zusätzliche Stabilisierungsmaßnahmen ausgebildet ist;
**dass** der sich an den Sprunggelenkbereich (2) anschließende Wadenbereich (1) nach vorn gewölbt ausgebildet ist, einen zusätzlichen Stabilisierungskern (1.1) aufweist und jeweils seitlich weitestgehend flexible und der Wadenform und -größe des Trägers angepasste, vorgeformt gewölbte Flügelschalen (1.2) angeordnet sind.

2. Stützfeder für den Einbau in eine Unterschenkelorthese nach dem Anspruch 1, **dadurch gekennzeichnet, dass** als Verstärkungsbereich mindestens eine Stabilitätssicke (3.3.1) im unteren Fersenbereich (3.3) längs verlaufend unteren Fersenbereiches 3.3 angeordnet ist.

3. Stützfeder für den Einbau in eine Unterschenkelorthese nach den Ansprüchen 1 und 2, **dadurch gekennzeichnet, dass** im Mittelpunkt der 3D-Fersenform (3.1) eine wenigstens ein Drittel der Fersenformgröße aufweisende Ausnehmung (3.2) ausgebildet ist.

4. Stützfeder für den Einbau in eine Unterschenkelorthese nach einem der Ansprüche 1 bis 3, **dadurch gekennzeichnet, dass** die Querschnittsform zu beiden Enden der Stützfeder gegen Null ausläuft, indem sich die Materialstärke der Stützfeder als sich verjüngend ausgebildet ist.

5. Stützfeder für den Einbau in eine Unterschenkelorthese nach einem der Ansprüche 1 bis 4, **dadurch gekennzeichnet, dass** die Stützfeder aus einem Faser-Kunststoff-Verbund ausgebildet ist, der aus einem textilen Halbzeug besteht, welches in einm Kunststoff eingebettet ist und bei dessen Aushärtung gleichzeitig die gewünschte 3D-Formgebung aufweist,
dass der Faser-Kunststoff-Verbund aus einer biegeschlaffen textilen ebenflächigen Trägermatte besteht, auf der vor der Einbettung in die Harzinfusion oder Kunststoff Verstärkungsfasern bedarfsweise richtungsorientiert fixiert sind und
dass die so gebildete Verstärkungsstruktur im gesamten Bereich des die Stützfeder bildenden Faser-Kunststoff-Verbundes ausgebildet ist, wobei das textile Halbzeug ein- oder mehrlagig angeordnet ist.

6. Stützfeder für den Einbau in eine Unterschenkelorthese nach einem der Ansprüche 1 bis 5, **dadurch gekennzeichnet, dass** die eingesetzten Verstärkungsfasern aus anorganischen, organischen und Naturfasern und/oder einer Kombination aus diesen Fasern bestehen.

7. Stützfeder für den Einbau in eine Unterschenkelorthese nach einem der Ansprüche 1 bis 6, **dadurch gekennzeichnet, dass** die biegeschlaffe textile ebenflächige Trägermatte aus einem Gewebe aus anorganischen, organischen und/oder Naturfasern und/oder einer Kombination aus diesen Fasern besteht.
